# EUROPEAN PATENT APPLICATION

(11) **EP 4 591 866 A1**
(43) Date of publication of application: **30.07.2025**
(21) Application number: 23867631.6
(22) Date of filing: 22.09.2023
(51) Int. Cl.: A61K 31/675, A61K 31/664, A61K 31/06, A61P 35/00

(54) **USE OF HYPOXIA-ACTIVATED COMPOUND IN PREPARATION OF MEDICAMENT FOR TREATING CANCER PATIENT**

(30) Priority: 22.09.2022 CN 202211158936
(71) Applicant: Ascentawits Pharmaceuticals, Ltd., Shenzhen, Guangdong 518122 (CN)
(72) Inventor: QI, Tianyang, Shenzhen, Guangdong 518000 (CN); MENG, Fanying, San Francisco California 94121 (US); DUAN, Jianxin, Shenzhen, Guangdong 518000 (CN)
(74) Representative: Haseltine Lake Kempner LLP
(86) International application number: PCT/CN2023/120712
(87) International publication number: WO 2024/061346

(57) **Abstract**

Provided is use of a hypoxia-activated compound alone or in combination with a PARP inhibitor in the preparation of a medicament for treating a cancer patient. In particular, the patient is detected to have pathogenic gene mutations such as an RAD51D mutation. At the same time, a method for treating cancer, a drug combination, and a pharmaceutical preparation are provided. The method, the drug combination, and the pharmaceutical preparation have a very good tumor inhibition effect on the patient with pathogenic gene mutations such as an RAD51D mutation when administered alone, and have a good tumor inhibition effect even if the patient has resistance to the PARP inhibitor. The drug used in combination with PRAP has a certain synergistic effect compared with the efficacy of the drug alone.

## Description

### Technical Field

The present invention relates to use of a hypoxia-activated compound alone or in combination with a PARP inhibitor (PARPi) in the treatment of a disease caused by pathogenic gene mutation of homologous recombination, and in particular to use in RAD51 pathogenic mutation. Also provided are a corresponding treatment method, a pharmaceutical combination, and a pharmaceutical preparation. The present invention is in the field of biomedicine.

### Background Art

Various endogenous and exogenous factors such as reactive oxygen species resulting from cell metabolism, ultraviolet ray, and genotoxic chemicals can constantly induce DNA damage. DNA damage can be roughly categorized into base damage, DNA cross-linking, single-strand breaks (SSBs), and double-strand breaks (DSBs), wherein DNA double-strand breaks are the deadliest DNA damage, which, without timely and correct repair, can cause chromosome deletion, fusion, and translocation, leading to cell death or even tumor occurrence and progression. Homologous recombination (HR) is the primary mechanism for the repair of DNA double-strand.

DNA damage repair involves a complex signaling network under strict control, comprising damage monitoring, short cycle regulation, and DNA repair to finally complete the whole process, wherein multiple proteins for example PARP, ATR, ATM, Wee1, DNA-PK, BRCA, RAD51, and PRMT5 play an important role in this process.

RAD51 gene encodes a polypeptide with 339 amino acids and a relative molecular weight of 4.3 × 10⁴, and has 5 homologs, XRCC2, XRCC3, RAD51B (RAD51L1), RAD51C (RAD51L2), and RAD51D (RAD551L3), which together with RAD51 are involved in HR process.

PARP inhibitors can inhibit the DNA single-strand damage repair function of PARP, leading to a great amount of single-strand DNA damage without timely repair in cells. Unrepaired single-strand DNA damage will initiate replication fork collapse and hence cause double-strand DNA damage. Double-strand DNA damage with higher cytotoxicity can be repaired in normal cells through the homologous recombination (HR) repair pathway that is mediated jointly by BRCA, RAD51, and other proteins.

Populations enrolled in the initial studies of PARPis for ovarian cancer all had previously received platinum chemotherapy, and research has found that platinum sensitivity directly correlates with response to PARPis (platinum chemotherapeutic drugs are DNA damaging agents and may cause DNA cross-linking that are partially repairable via the HR pathway; therefore, DNA repair deficient tumors are expected to be sensitive to platinum chemotherapy). Two PARPis have been approved for ovarian cancer: Niraparib and Rucaparib. Niraparib is approved by FDA and EMA for a maintenance regimen, regardless of BRCA1/2 status. Rucaparib is also registered by the FDA and EMA as optional treatment regimen for BRCA1/2-mutation-associated ovarian cancer patients who have received two previous lines of chemotherapy. In addition, talazoparib has also been approved by FDA for treatment of BRCA-mutated/HER2-negative metastatic breast cancer (Mateo, J., Lord, C. J., Serra, V., Tutt, A., Balmaña, J., Castroviejo-Bermejo, M., Cruz, C., Oaknin, A., Kaye, S. B., & de Bono, J. S. (2019). A decade of clinical development of PARP inhibitors in perspective. Annals of oncology: official journal of the European Society for Medical Oncology, 30(9), 1437-1447. https://doi.org/10.1093/annonc/mdz192).

In prior art WO2019133697A1 (corresponding to the Chinese Application CN111801117A), the PARP inhibitor Niraparib used by researchers could benefit patients with homologous recombination repair (HRR) deficiency (not of BRCA1 or BRCA2). With the clinical use of PARPis, PARPi resistance has become an inevitable problem therein. Studies to date have demonstrated that the main causes of PARPi resistance include homologous recombination repair (HRR), DNA replication fork protection, and pharmacokinetic changes of PARPi. In order to overcome PARPi resistance and increase sensitivity to PARPi drugs, various combination therapies are being developed, many of which have entered clinical phases. The various combination therapies mainly include PARPi-DNA alkylating agent combination, PARPi-oncolytic herpes simplex virus (oHSVs) combination, PARPi-ionizing radiation combination, PARPi-immunotherapy combination, PARPi-HSP90 inhibitor combination, PARPi-WEE1/ATR inhibitor combination, PARPi-DNMTi inhibitor combination, PARPi-CDK inhibitor combination, etc. (He Li, Zhao-Yi Liu, Nayiyuan Wu,Yong-Chang Chen, Quan Cheng and Jing Wang. PARP inhibitor resistance: the underlying mechanisms and clinical implications. Mol Cancer, 2020 Jun 20;19(1):107.2020. https://doi.org/10.1186/s12943-020-01227-0; Rose, M., Burgess, J. T., O'Byrne, K., Richard, D. J., & Bolderson, E. (2020). PARP Inhibitors: Clinical Relevance, Mechanisms of Action and Tumor Resistance. Frontiers in cell and developmental biology, 8, 564601. https://doi.org/10.3389/fcell.2020.564601).

TH-302 (evofosfamide, CAS NO. 918633-87-1) and analogs thereof are 2-nitroimidazole-initiated hypoxia-activated prodrug (HAP) bromoisophosphoramide developed by Threshold Pharmaceuticals, Inc., the United States of America. In the case of hypoxia, the inactive TH-302 prodrug can release highly toxic Br-IPM. TH-302 has a broad spectrum of biological activities in vitro and in vivo and a specific hypoxia-selective activation activity, and induces γH2AX phosphorylation and DNA cross-linking, thereby leading to cell cycle arrest and death. Thus, this compound has been used for the development of anticancer drugs by several pharmaceutical companies and scientific research institutes.

As pointed out in a research document by Meng F Y (Meng Fanying) et al., TH-302 shows broad-spectrum activity against various tumors and provides an excellent hypoxia-selective activity-enhancing effect. The study has shown that TH-302 exhibits significantly higher *in vitro* cytotoxicity to 32 human cancer cell lines under hypoxia than under normoxia, demonstrating hypoxia-selective cytotoxicity of this compound to cancer cells under hypoxic conditions. With human cells overexpressing single-electron reductase (POR), the mechanism of one-electron-reductase-dependent activity enhancement of TH-302 under hypoxia has been confirmed, as represented by Reaction Equation 1 below:

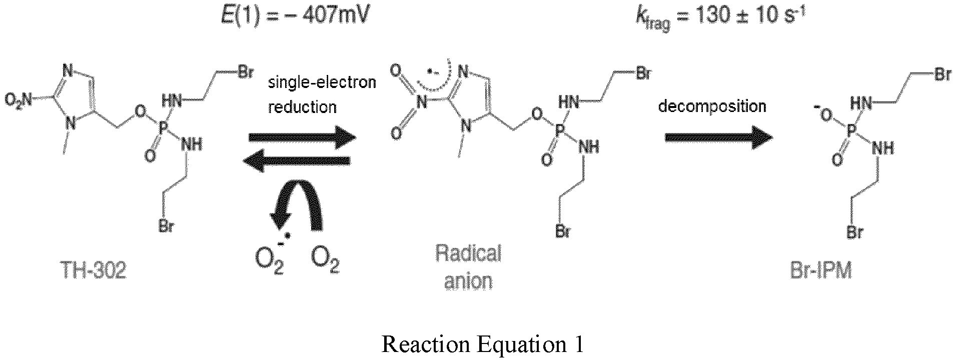

Cytochrome P450 oxidoreductase reduces the prodrug TH-302 into an intermediate radical anion, which is unstable and decomposes into the cytotoxin Br-IPM with cytotoxicity to play its role. The key step of this process is the single-electron reduction process. Studies have confirmed that the presence of oxygen will reverse the single-electron reduction process. In other words, the presence of oxygen will hinder the single-electron reduction process. Therefore, only under anoxia/hypoxia, TH-302 can be reduced to produce greater cytotoxicity.

The applicant made modifications to TH-302, wherein the phosphoramidate moiety was modified into an aziridine structure and the nitroimidazole moiety was modified into a fused ring structure containing a nitrobenzene ring (patent application PCT/CN2020/114519 with publication number WO2021120717A1), similarly obtaining a broad-spectrum anticancer prodrug with hypoxia-activated activity.

### Summary of the Invention

Researchers of the applicant found in pharmacodynamic experiments that hypoxia-activated TH-302 and aziridine structural analogs thereof with structural modifications (patent application PCT/CN2020/114519 with publication number WO2021120717A1) have a very good tumor inhibition effect on a patient with pathogenic RAD51D gene mutation when administered alone, and have a good tumor inhibition effect even if the patient has resistance to PARP inhibitors (PARPis). Further research found that each of the above-mentioned hypoxia-activated TH-302 and aziridine structural analogs thereof with structural modifications used in combination with a PARPi has a certain synergistic effect compared with the efficacy of themselves alone.

Accordingly, the present application provides the following method for treating cancer, pharmaceutical use, pharmaceutical combination, and corresponding pharmaceutical preparation.

Provided is a treatment method for treating patients with cancer or tumors with impaired DNA repair enzymes, comprising administering a hypoxia-activated compound, or a salt, ester, solvate, isotopic variant, or isomer thereof either as monotherapy or in combination with other drugs.

Specifically, the DNA repair enzyme impairment is selected from defects in one or more of the following genes: BRCA1, BRCA2, FANCA, FANCD1, FANCD2, ATM, ATR, CHEK1, CHEK2, CTP, BARD1, BRIP1, PALB2, RAD51, RAD52, RAD54, RAD55, RAD57, FAM175, NBN, Rad50, MRE11, p53, NBS1, XRS2, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RBBP8, SMC5, USP11, and WRN; the RAD51 is selected from XRCC2, XRCC3, RAD51B, RAD51C, and RAD51D, and preferably RAD51D.

In one embodiment, the DNA repair enzyme impairment is selected from one or more of:
homologous recombination DNA repair enzyme impairment,
nucleotide excision repair enzyme impairment,
nonhomologous end joining enzyme impairment,
base excision repair enzyme impairment,
mismatch repair enzyme impairment, and
Fanconi's anemia pathway repair enzyme impairment.

More preferred is any one or more of homologous recombination DNA repair enzyme impairment, nucleotide excision repair enzyme impairment, and base excision repair enzyme impairment. Further preferred is homologous recombination DNA repair enzyme impairment alone or both homologous recombination DNA repair enzyme impairment and nucleotide excision repair enzyme impairment.

Specifically, the above-mentioned other drugs include a PARP inhibitor which is selected from Olaparib, Rucaparib, Niraparib, Talazoparib, Fluzoparib, and Pamiparib.

Specifically, the above-mentioned cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.

Specifically, the patient is resistant or insensitive to the above-mentioned PARP inhibitor.

Sensitivity and resistance are qualitative assessments used to determine whether a patient exhibits resistance to a drug. They are often seen in drug sensitivity tests of microbial pathogens and antimicrobial (antibacterial and antifungal) agents.

Sensitivity and resistance are two different results shown in a clinical drug sensitivity test. The sensitivity means that, in a drug sensitivity test, a pathogen is found to be sensitive to a drug, i.e. the drug can completely kill the pathogen; while the resistance means the drug is found to be unable to kill the pathogen in the test, i.e. the drug is ineffective. Clinically, obtaining data on whether or not a drug is resistant is of great significance to the treatment of diseases, as it allows for the selection of more sensitive drugs to kill the pathogen. Aside from sensitivity and resistance, another condition is "morderate sensitivity", meaning "moderately Susceptible", indicating easy development of resistance in spite of possible effectiveness and expected inferior efficacy compared with treatment to which the pathogen is susceptible.

In a chemotherapy (compared to surgery and radiotherapy, in a broad sense to mean the use of a drug to treat cancer) regimen for cancer treatment, according to clinical treatment guidelines, typically a series of chemotherapeutic drugs are administered in sequence. When the drug first administered has no or minimal therapeutic effect on the patient after a period of administration, the cancer type of the patient is considered to have developed tolerance, resistance, or insensitivity to the drug, where a further drug (second-line drug) is needed. Likewise, tolerance, resistance, or insensitivity may be again developed to the second-line drug after a period of administration, where a third-line drug is needed. Sensitivity and resistance are diagnosed and determined by medical professionals or clinicians.

Apparently, the specific meaning of the cancer or tumors patient being sensitive to PARP inhibitors is that there is an effect after the administration of PARP inhibitors or it is judged by the doctor's diagnosis and treatment that PARP inhibitors will be effective.

In one embodiment of the treatment method, it uses a hypoxia-activated compound, or a salt, ester, solvate, isotopic variant, or isomer thereof alone to treat a patient with cancer or tumors caused by RAD51D gene defect, wherein the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

In one embodiment of the treatment method, it uses a hypoxia-activated compound, or a salt, ester, solvate, isotopic variant, or isomer thereof in combination with a PARP inhibitor to treat a patient with cancer or tumors caused by RAD51D gene defect, wherein the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer; and the PARP inhibitor is selected from Olaparib, Rucaparib, Niraparib, Talazoparib, Fluzoparib, and Pamiparib.

Specifically, the patient is resistant or insensitive to the PARP inhibitor.

Regarding the medicament described herein, it refers to a medicinal product or preparation. The prepared medicinal product contains a specific dosage range of a hypoxia-activated compound, or a salt or solvate thereof as an active ingredient, and/or the prepared medicament is in a particular dosage form and is administered via a particular mode of administration.

The prepared medicinal product, medicament, or preparation may further comprise pharmaceutically acceptable auxiliaries or excipients. The medicament may be in any dosage form for clinical administration, such as tablets, suppositories, dispersible tablets, enteric tablets, chewable tablets, orally disintegrating tablets, capsules, sugar-coated preparations, granules, dry powders, oral solutions, small dose injections, lyophilized powder for injection, or infusion solutions. According to the specific dosage form and the mode of administration, the pharmaceutically acceptable auxiliaries or excipients in the medicament may include one or more of diluents, solubilizers, disintegrants, suspending agents, lubricants, binding agents, fillers, flavoring agents, sweeteners, antioxidants, surfactants, preservatives, wrapping agents, and pigments, etc.

Specifically, in the above-mentioned treatment method, the hypoxia-activated compound is selected from the compound of Formula I or II below: 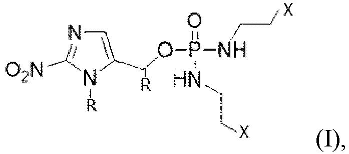
wherein Rs are each independently selected from H, -CH₃, and -CH₂CH₃, and Xs are each independently selected from leaving functional groups such as Cl, Br, MsO, and TsO; 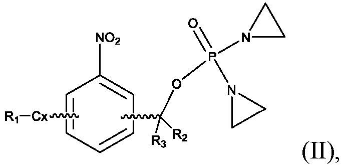
wherein the definitions of R₁, R₂, R₃, and Cx are as described in the claims of patent application PCT/CN2020/114519 with publication number WO2021120717A1, which is incorporated herein by reference in its entirety.

A compound of Formula II obtained by modification of the structure of TH-302 has the same hypoxia activation mechanism: it has higher cytotoxicity to cancer cells under hypoxia than under normoxia. A compound of Formula II is not a P-gp substrate, and thus can effectively enter the central nervous system such as brain, can accumulate to a concentration high enough to exert its effect on the central nervous system, and is expected to become a drug for treating primary brain cancer, tumor or metastatic cancer or tumors that metastasizes to the brain or to be used therefor. A compound of Formula II is relatively stable in human serum and liver microsomes and has the potential to be developed into a drug. Researchers further demonstrated through *in vivo* animal experiments that: a compound of Formula II has a higher MTD, i.e. the compound with a new structure, as compared with the compound disclosed in the prior literature, has higher safety and clinical treatment dosage and possibly better therapeutic effect on cancer or tumors. For comparison, its effect on inhibiting tumor growth is significantly superior to that of the classic chemotherapeutic drug Ifosfamide (also a drug with the DNA alkylating agent mechanism).

Specifically, the definitions of R₁, R₂, R₃, and Cx are as follows:
Cx is a 5-10-membered aromatic ring or aromatic heterocycle, aliphatic heterocycle or cycloalkane, which shares two carbon atoms with a nitrobenzene ring to form a fused ring structure;
R₁ linked to any backbone atom on the Cx ring is selected from the group consisting of hydrogen, a halogen atom, cyano or isocyano, hydroxyl, sulfhydryl, amino, OTs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, alkoxyl with 1-6 carbon atoms or Z-substituted alkoxyl with 1-6 carbon atoms, -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷, and - NR⁶SO₂NR⁶R⁷,
R₂ and R₃ are each independently hydrogen, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, or 5-15-membered heteroaryl or Z-substituted heteroaryl, or R₂ and R₃ together with the benzylic carbon atom to which they are bonded form a 3-6-membered ring; 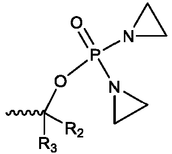
group can substitute a hydrogen atom at any position of the fused ring carbon atoms, and the number of substitution is 1;
the substituent Z is a halogen atom, cyano or isocyano, hydroxyl, sulfhydryl, amino, C₁-C₃ alkyl or substituted alkyl, C₁-C₃ alkoxyl or substituted alkoxyl, C₂-C₃ alkenyl or substituted alkenyl, C₂-C₃ alkynyl or substituted alkynyl, C₃-C₈ cycloalkyl or substituted cycloalkyl;
R⁶ and R⁷ are each independently hydrogen, C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4-15-membered heterocyclyl or Z-substituted 4-15-membered heterocyclyl, or 5-15-membered heteroaryl or Z-substituted 5-15-membered heteroaryl, or R⁶ and R⁷ together with the atom to which they are bonded form a 5-7-membered heterocyclyl or Z-substituted 5-7-membered heterocyclyl.

Specifically, the compound of Formula I is selected from the compounds of the following structures: 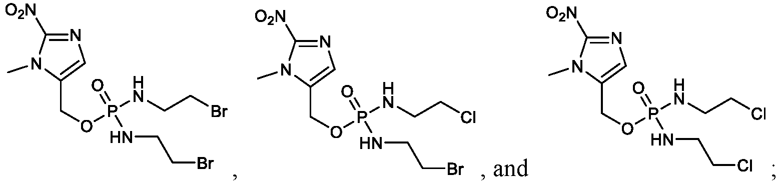 specifically, the compound of Formula II is selected from the compounds of the following structures:

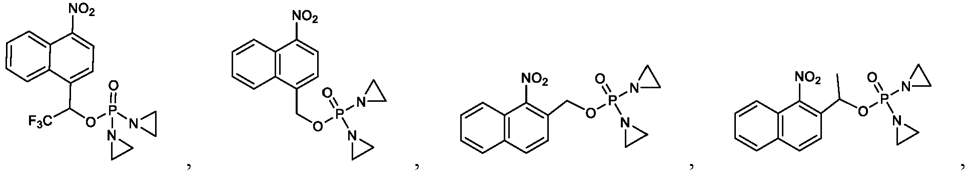

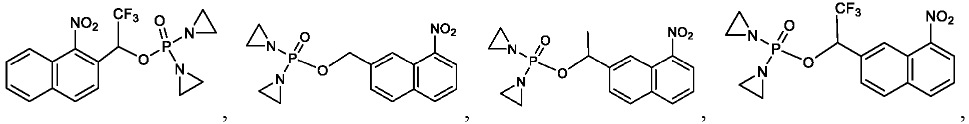

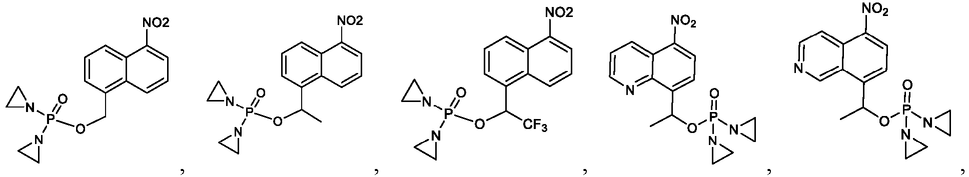

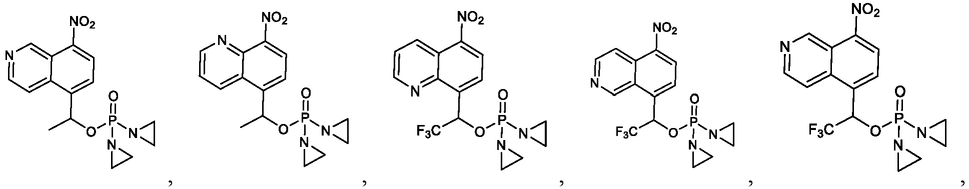

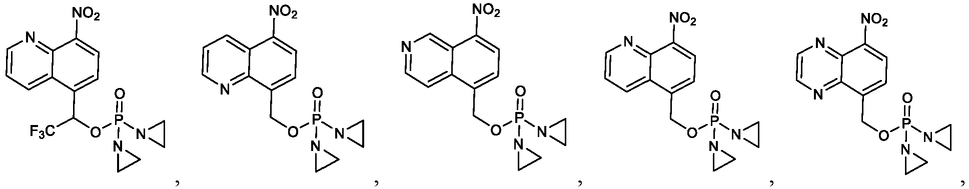

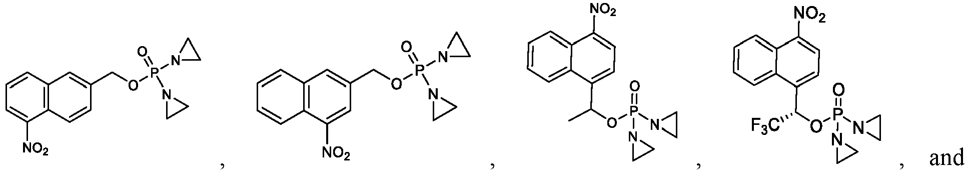

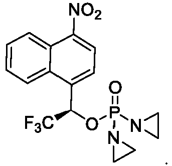

Preparations relevant to TH-302 or similar compounds of Formula I thereto include oral preparations, lyophilized preparations, and concentrated injections; and the relevant formulations, preparation methods and clinical compatibility, and modes of administration have been described in detail and disclosed by the relevant patents of Threshold Pharmaceuticals, Inc.: WO2010048330A1, WO2012142520A2, and WO2008083101A1; the above patent applications are incorporated herein by reference in their entirety.

TH-302 or a similar compound of Formula I thereto is a DNA alkylating anticancer drug with extensive cancer treatment potential. Experiments on cancer indications and clinical trials relevant to TH-302 have been disclosed in relevant patent applications of Threshold Pharmaceuticals, Inc. and other pharmaceutical companies, such as WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, and WO2015051921A2), and clinical trials registered with the FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, and NCT02712567); the above relevant applications and clinical trial information are incorporated herein by reference in their entirety.

"Cancer" refers to leukemia, lymphoma, carcinoma, and other malignant tumors (including solid tumors) with potentially unrestrained growth that can expand locally by invasion and systemically by metastasis.

Listed here are examples of cancer treatable with TH-302 or similar compounds of Formula I thereto, including, but not limited to, cancers of the adrenal gland, bone, brain, breast, bronchus, colon and/or rectum, gallbladder, head and neck, kidney, larynx, liver, lung, nervous tissue, pancreas, prostate, accessory thyroid gland, skin, stomach, and thyroid gland. Certain other examples of cancer include acute and chronic lymphocytic and granulocytic neoplasms, adenocarcinoma, adenoma, basal cell carcinoma, cervical epithelial dysplasia and carcinoma *in situ,* Ewing's sarcoma, epidermoid carcinoma, giant cell tumor, glioblastoma multiforme, pilocytic tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcoma, osteogenic and other sarcomas, ovarian tumor, pheochromocytoma, polycythaemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesion, reticulum cell sarcoma, and Wilm's tumor.

PARP is an enzyme, whose full name is Poly ADP-ribose Polymerase. It is a DNA repair enzyme that plays a crucial role in DNA repair pathways. PARP is activated in response to DNA single-strand damage or breakage. As a molecular sensor for DNA damage, it has the function to identify and bind to DNA break sites and thus activate and catalyze poly(ADP-ribosyl)ation of receptor proteins to get involved in DNA repair.

PARP inhibitors stop the PARP enzyme corresponding to a "repairer" from working properly by inhibiting its function. Without repair to DNA damage, cells may die. PARP inhibitors are compounds with inhibitory effect on the PARP enzyme, i.e. any substance that can inhibit the activity of the PARP enzyme is a PARP inhibitor.

Specifically, the PARP inhibitor of the present application is selected from the group consisting of ABT-767, AZD 2461, BGB-290, BGP 15, CEP 8983, CEP 9722, DR2313, E7016, E7449, Fluzoparib, IMP 4297, INO1001, JPI 289, JPI 547, monoclonal antibody B3-LysPE40 conjugate, MP 124, Niraparib, NU 1025, NU 1064, NU 1076, NU1085, Olaparib, ONO2231, PD 128763, R 503, R554, Rucaparib, SBP 101, SC 101914, Simmiparib, Pamiparib, Talazoparib, Veliparib, WW 46, 2-(4-(trifluoromethyl)phenyl)-7,8-dihydro-5*H*-thiofuro[4,3-*d*]pyrimidin-4-ol, and salts or derivatives thereof.

Preferably, the PARP inhibitor is selected from the 5 drugs already commercially available, namely Olaparib, Rucaparib, Niraparib, Talazoparib, and Fluzoparib, and the drug in a Phase III clinical trial, Pamiparib. Clearly, the PARP inhibitor herein essentially refers to a drug containing a PARP inhibitor active ingredient.

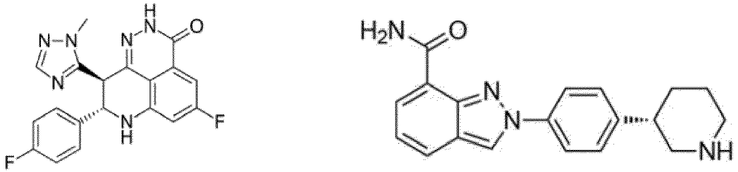

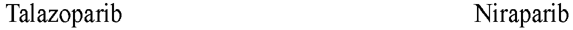

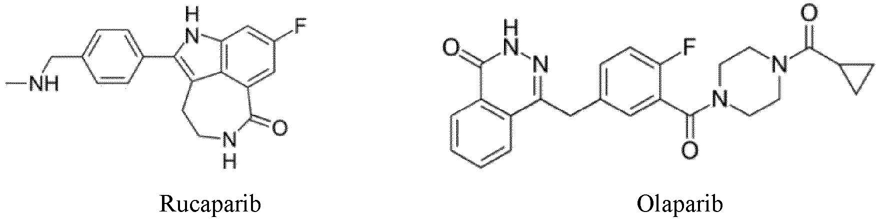

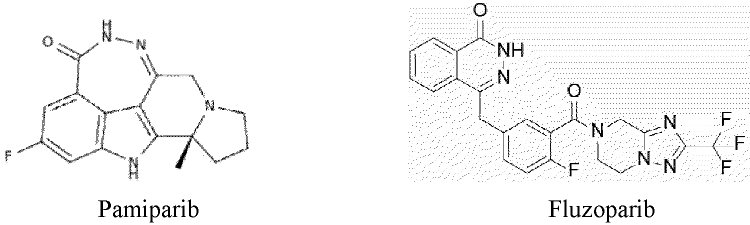

Talazoparib is indicated for adults with deleterious or suspected deleterious germline BRCA-mutated (gBRCAm) HER2-negative locally advanced or metastatic breast cancer. The commercially available dosage form is the 0.25 mg/1 mg talazoparib tosylate capsule. 1 mg is taken orally once daily. In the event of adverse reactions, consider interruption of treatment or dose reduction:
for the first adverse reaction, reduce the oral dosage to 0.75 mg (three 0.25 mg capsules) once daily;
for the second adverse reaction, reduce the oral dosage to 0.5 mg (two 0.25 mg capsules) once daily; and
for the third adverse reaction, reduce the oral dosage to 0.25 mg (one 0.25 mg capsule) once daily.

Niraparib is used for the maintenance treatment of adult patients with platinum-sensitive recurrent epithelial ovarian, fallopian tube, or primary peritoneal cancer that has achieved complete or partial remission after platinum-based chemotherapy. The commercially available dosage form is the 100 mg niraparib tosylate capsule. 300 mg is taken orally once daily until the occurrence of disease progression or unacceptable adverse reaction. In the event of adverse reactions, consider interruption of treatment or dose reduction:
the first dose reduction is from three capsules (300 mg) per day to two capsules (200 mg) per day; if further dose reduction is required, the second dose reduction is from two capsules (200 mg) per day to one capsule (100 mg) per day; and
if dosing interruption and dose reduction cannot control adverse reactions, dose discontinuation is recommended.

Rucaparib is used for females with advanced ovarian cancer that carries a specific gene mutation (deleterious BRCA mutation) who have been treated with two or more chemotherapeutic drugs. The commercially available dosage form is tablets in three specifications: 200 mg, 250 mg, and 300 mg. Recommended dosage is 600 mg orally twice daily with or without food. Continue treatment until the occurrence of disease progression or unacceptable toxicity. In the event of adverse reactions, consider interruption of treatment or dose reduction.

Olaparib is used for the maintenance treatment of adult patients with germline or somatic BRCA-mutated (gBRCAm or sBRCAm) advanced epithelial ovarian, fallopian tube or primary peritoneal cancer that has achieved complete or partial remission after the first-line platinum-based chemotherapy; and for the maintenance treatment of adult patients with platinum-sensitive recurrent epithelial ovarian, fallopian tube or primary peritoneal cancer that has achieved complete or partial remission after platinum-based chemotherapy. The commercially available dosage form is tablets in two specifications: 150 mg and 100 mg. Recommended dosage is 300 mg (two 150 mg tablets) taken twice daily, equivalent to a total daily dose of 600 mg. 100 mg tablets are for use in dose reduction, and in the event of adverse reactions, e.g. nausea, vomiting, diarrhea, and anemia, consider interruption of treatment or dose reduction:
if dose reduction is required, the recommended dosage is reduced to 250 mg (one 150 mg tablet and one 100 mg tablet) taken twice daily (equivalent to a total daily dose of 500 mg); and
if further dose reduction is required, then the recommended dosage is reduced to 200 mg (two 100 mg tablets) taken twice daily (equivalent to a total daily dose of 400 mg).

Fluzoparib is used for the treatment of patients with germline BRCA-mutated (gBRCAm) platinum-sensitive recurrent ovarian, fallopian tube or primary peritoneal cancer who have been treated with two or more chemotherapies. The commercially available dosage form is capsules in the specification of 50 mg.

For other PARPi candidates under development which have entered clinical trials, see the web link https://www.selleckchem.com/PARP.html and related academic reviews.

For the recommended dosage of TH-302 or similar compounds of Formula I thereto for treating cancer, see dosages in patent applications by Threshold Pharmaceuticals, Inc. and other pharmaceutical companies (e.g. WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, and WO2015051921A2) and clinical trials registered with the FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, and NCT02712567):
a daily dose of 120 mg/m² to 460 mg/m² for intravenous injection administration;
a weekly dose of 480 mg/m² to about 670 mg/m² or, for example, 575 mg/m² for intravenous injection administration.

TH-302 (concentrate of solution for administration) for use in clinical trials is a sterile liquid formulation of TH-302. TH-302 is formulated with 70% anhydrous ethanol, 25% dimethlyacetamide, and 5% polysorbate 80. It is supplied by the sponsors in a 10 mL glass vial with a rubber stopper and flip-off seal. TH-302 drug product is a clear, colorless to light yellow solution, essentially free of visible particulates. Each single-use vial contains a nominal fill volume of 6.5 mL of TH-302 drug product for a nominal total of 650 mg of TH-302 (equivalent to 100 mg/mL) and is labeled clearly, disclosing the batch number, route of administration, required storage conditions, sponsor's name, and appropriate precautionary labeling as required by applicable regulations. Dilution prior to administration is required per the pharmacy manual.

TH 302 drug product is diluted prior to administration with commercially available 5% glucose in water to a total volume of 500 mL (1000 mL for a total dose of ≥ 1000 mg) to obtain the desired final concentration. Each dose of TH 302 is prepared with 5% glucose in water without di(2-ethylhexyl)phthalate (non-DEHP), and administered by intravenous drip via a non-DEHP intravenous infusion administration set.

Lyophilized preparations developed by Threshold Pharmaceuticals, Inc. can also be used:
A solution (20 mL) of TH-302 (100 mg) and sucrose (1 g) is added to a lyophilization vial and lyophilized to produce a lyophilized unit dosage form of TH-302 with a drug load of less than 5 mg/cm³. For the purpose of human administration, the unit dosage form is dissolved in 5% glucose injection and an appropriate amount of the solution is administered to a patient.

The subsequent Phase I clinical trial dosage regimen of TH-302 in human patients uses lyophilized preparations. TH-302 lyophilized preparation for injection is prepared in a 100 mL glass vial with a drug load of 100 mg/100 mL, and stored under controlled conditions at 2-8 °C. 250 mL of 5% glucose injection is injected into the lyophilized preparation vial for use, and an intravenous drip is performed through an infusion pump within 30 minutes.

Monotherapy refers to single drug therapy. Combination therapy refers to combined drug therapy. Single drug therapy refers to the use of only one anticancer drug in a course of treatment. Combination therapy refers to the simultaneous or sequential use of two or more anticancer drugs in a course of treatment.

Generally speaking, for combination therapy, different administration dosages and administration cycles need to be explored according to the characteristics of the disease and the types of drugs to be used in combination; and only the combined drug therapy regimen obtained from the exploration according to the above conditions may achieve better therapeutic effect as compared with single drug therapy.

The administration dosages and administration cycles of drugs in both monotherapy and combination therapy regimens need to be explored through clinical trials with the reference to the dosages and dosage regimens of the above-mentioned TH-302 and similar compounds thereto and the PARPi.

The present application further provides the steps of the above-mentioned treatment method, comprising:
detecting RAD51D gene mutations in patients with cancer or tumors;
if the patient or the tumor tissue thereof has pathogenic RAD51D gene mutation, using a medicament containing the hypoxia-activated compound of Formula I or II below alone or in combination with a PARP inhibitor for treatment: 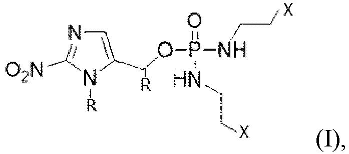
wherein Rs are each independently selected from H, -CH₃, and -CH₂CH₃, and Xs are each independently selected from leaving functional groups such as Cl, Br, MsO, and TsO; 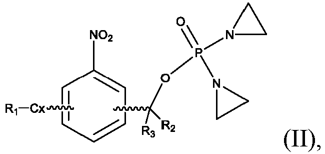
wherein the definitions of R₁, R₂, R₃, and Cx are described in the claims of patent application PCT/CN2020/114519 with publication number WO2021120717A1, which is incorporated herein by reference in its entirety.

Specifically, the definitions of R₁, R₂, R₃, and Cx are as follows:
Cx is a 5-10-membered aromatic ring or aromatic heterocycle, aliphatic heterocycle or cycloalkane, which shares two carbon atoms with a nitrobenzene ring to form a fused ring structure;
R₁ linked to any backbone atom on the Cx ring is selected from the group consisting of hydrogen, a halogen atom, cyano or isocyano, hydroxyl, sulfhydryl, amino, OTs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, alkoxyl with 1-6 carbon atoms or Z-substituted alkoxyl with 1-6 carbon atoms, -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷, and - NR⁶SO₂NR⁶R⁷,
R₂ and R₃ are each independently hydrogen, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, or 5-15-membered heteroaryl or Z-substituted heteroaryl, or R₂ and R₃ together with the benzylic carbon atom to which they are bonded form a 3-6-membered ring; 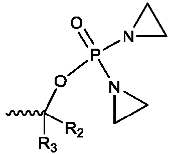
group can substitute a hydrogen atom at any position of the fused ring carbon atoms, and the number of substitution is 1;
the substituent Z is a halogen atom, cyano or isocyano, hydroxyl, sulfhydryl, amino, C₁-C₃ alkyl or substituted alkyl, C₁-C₃ alkoxyl or substituted alkoxyl, C₂-C₃ alkenyl or substituted alkenyl, C₂-C₃ alkynyl or substituted alkynyl, C₃-C₈ cycloalkyl or substituted cycloalkyl;
R⁶ and R⁷ are each independently hydrogen, C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4-15-membered heterocyclyl or Z-substituted 4-15-membered heterocyclyl, or 5-15-membered heteroaryl or Z-substituted 5-15-membered heteroaryl, or R⁶ and R⁷ together with the atom to which they are bonded form a 5-7-membered heterocyclyl or Z-substituted 5-7-membered heterocyclyl.

Specifically, the TMB (Tumor mutation burden) level of the aforementioned RAD51D gene mutation is moderate.

Due to differences in TMB (Tumor mutation load (burden)) level among different tumor types, it is generally acknowledged that, TMB levels that exceed 20 mutations/Mb (Mb represents every million bases) are considered high; those less than 10 mutations/Mb are considered low, while those in the middle are considered moderate. At the 2017 World Conference on Lung Cancer, Bristol-Myers Squibb Company (BMS) announced the results of a clinical trial called CheckMate-032. This is a phase II clinical trial that incorporated 401 patients with advanced lung cancer who had failed first-line treatment, and were treated with PD-1 inhibitors alone or in combination with ipilimumab. The patients were divided into three groups: high TMB, medium TMB and low TMB according to the TMB level. In the population receiving combination therapy, the effective rates of the three groups were 62%, 20%, and 23%, respectively, with the population with high TMB showing a 3-fold higher effectiveness rate. The median overall survival of the three groups was 22.0 months, 3.6 months, and 3.4 months, respectively - a 6-fold difference between 22.0 months and 3.4 months! The experiment demonstrates that different levels of TMB have a significant impact on the efficacy of different cancer treatment drugs.

Specifically, the PARP inhibitors are selected from Olaparib, Rucaparib, Niraparib, Talazoparib, Fluzoparib, and Pamiparib;
Specifically, the patient is resistant or insensitive to the aforementioned PARP inhibitors.

The present application also provides the pharmaceutical use of a hypoxia-activated compound of the following formula I or II, or a salt, ester, solvate, isotopic variant, or isomer thereof, and the said compounds are used in the manufacture of a medicament for treating patients with cancer or tumors with pathogenic RAD51D gene mutations either as monotherapy or in combination with other therapeutic drugs: 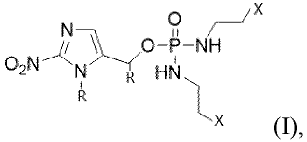
wherein, Rs are each independently selected from H-, CH₃, and -CH₂CH₃, and Xs are each independently selected from leaving functional groups such as Cl, Br, MsO, and TsO; 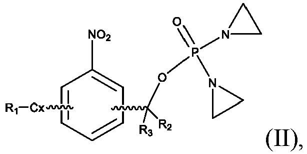
wherein, the definitions of R₁, R₂, R₃, and Cx are as described in the claims of patent application PCT/CN2020/114519 with publication number WO2021120717A1, which is incorporated herein in its entirety.

Specifically, for the aforementioned pharmaceutical uses, the TMB (Tumor mutation burden) level of the RAD51D gene mutation is moderate.

Specifically, for the aforementioned pharmaceutical uses, the other therapeutic drugs include a PARP inhibitor which is selected from Olaparib, Rucaparib, Niraparib, Talazoparib, Fluzoparib, and Pamiparib.

Specifically, for the aforementioned pharmaceutical uses, the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

Specifically, the patients are insensitive or resistant to the aforementioned PARP inhibitors.

The present application also provides a medicament for the treatment of the cancer caused by the pathogenic RAD51D gene mutation. The medicament comprises a hypoxia-activated compound of the following formula I or II, or a salt, ester, solvate, isotopic variant, or isomer thereof: 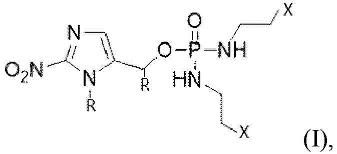
wherein, Rs are each independently selected from H-, CH₃, and -CH₂CH₃, and Xs are each independently selected from leaving functional groups such as Cl, Br, MsO, and TsO; 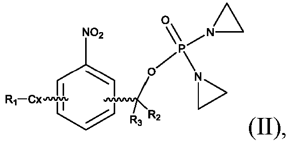
wherein, the definitions of R₁, R₂, R₃, and Cx are as described in the claims of patent application PCT/CN2020/114519 with publication number WO2021120717A1, which is incorporated herein in its entirety.

Specifically, for the aforementioned medicaments, when used, they may be used alone or in combination with PARP inhibitors.

Preferably, the PARP inhibitors are selected from Olaparib, Rucaparib, Niraparib, Talazoparib, Fluzoparib, and Pamiparib.

Preferably, the TMB (Tumor mutation burden) level of gene mutations is moderate.

Preferably, the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

Specifically, among the aforementioned drugs, the patient is resistant or insensitive to the PARP inhibitor.

The present application further provides a pharmaceutical combination, which comprises the following substances as active ingredients:
a hypoxia-activated compound, or a salt, ester, solvate, isotopic variant, or isomer thereof, and PARP inhibitors;
wherein the active ingredients are formulated together or separately, for use in compatible combination simultaneously or separately.

Generally, the active ingredients that are characteristic of the pharmaceutical combination described in the present application can be formulated either together (in a single dose unit) or separately (as a kit).

Generally, two active ingredients that are formulated together or separately may be administered simultaneously; or depending on the properties of their respective preparations, they are administered separately at certain time interval that is beneficial for optimizing their compatible effect.

The pharmaceutical combination may be a compound medicament, which is a pharmaceutical composition composed of an active ingredient that is the aforementioned hypoxia-activated compound, or a salt, ester, solvate, isotopic variant, or isomer thereof, and another active ingredient that is a PARP inhibitor. Under such circumstances, the aforementioned active ingredients are formulated together and administered simultaneously.

The pharmaceutical combination may also be a kit similar to Pfizer's medicament Paxlovid that acts against COVID-19, wherein the active ingredients may be sold simultaneously or separately. In this case, the aforementioned active ingredients are formulated separately. However, the two or more drugs that are formulated separately may be administered simultaneously, or be administered separately at certain time interval according to the properties of their respective preparations.

As a specific embodiment of the present invention where the active ingredients are formulated separately, one of the formulated medicament comprises the aforementioned hypoxia-activated compound, or a salt, ester, solvate, isotopic variant, or isomer thereof and pharmaceutically acceptable excipients, and the other medicament comprises PARP inhibitors and pharmaceutically acceptable excipients. The pharmaceutical combination composed of the two aforementioned medicaments may be administered simultaneously, or be administered separately at certain time interval according to the properties of their respective preparations.

The pharmaceutical combination provided in the present application is used for treating patients with cancer or tumors with DNA repair enzyme impairment. That is to say, the pharmaceutical combination may have better therapeutic effects on patients with cancer or tumors with DNA repair enzyme impairment.

Specially, the DNA repair enzyme impairment is selected from one or more of the following gene defects: BRCA1, BRCA2, FANCA, FANCD1, FANCD2, ATM, ATR, CHEK1, CHEK2, CTP, BARD1, BRIP1, PALB2, RAD51, RAD52, RAD54, RAD55, RAD57, FAM175, NBN, Rad50, MRE11, p53, NBS1, XRS2, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA Ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RBBP8, SMC5, USP11, and WRN; The RAD51 is selected from XRCC2, XRCC3, RAD51B, RAD51C, and RAD51D, preferably RAD51D.

Specially, the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.

As a preferred embodiment of the present application, the active ingredient PARP inhibitor in the pharmaceutical combination is selected from Olaparib, Rucaparib, Niraparib, Talazoparib, Fluzoparib, and Pamiparib; the active ingredient hypoxia-activated compound in the pharmaceutical combination is selected from the compounds of following formula I or II: 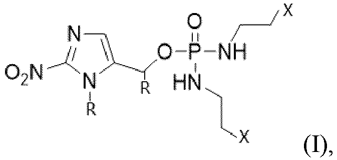
wherein, Rs are each independently selected from H, -CH3, and -CH2CH3, and Xs are each independently selected from leaving functional groups such as Cl, Br, MsO, and TsO; 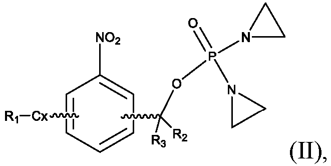
wherein, the definitions of R1, R2, R3, and Cx are as described in the claims of patent application PCT/CN2020/114519 with publication number WO2021120717A1.

As a preferred embodiment, the hypoxia-activated compound of formula I is selected from the following structures:

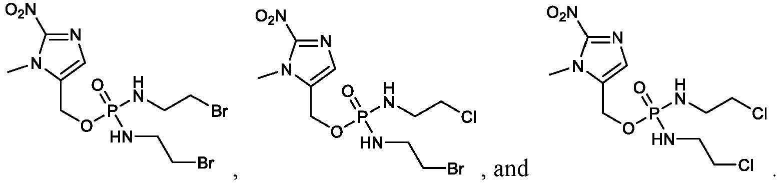

As a preferred embodiment, the hypoxia-activated compound of formula II is selected from the following structures:

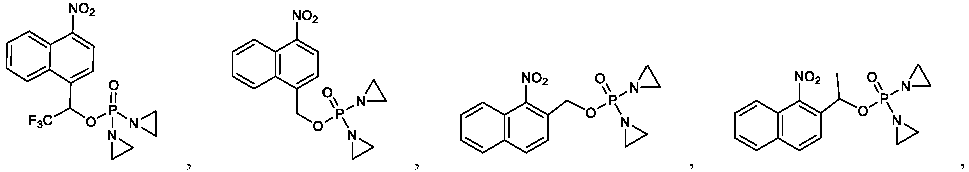

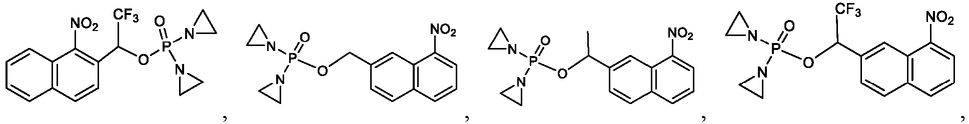

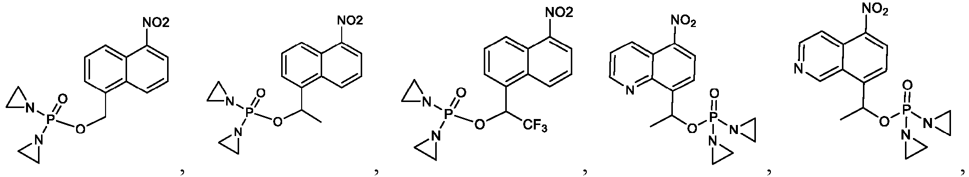

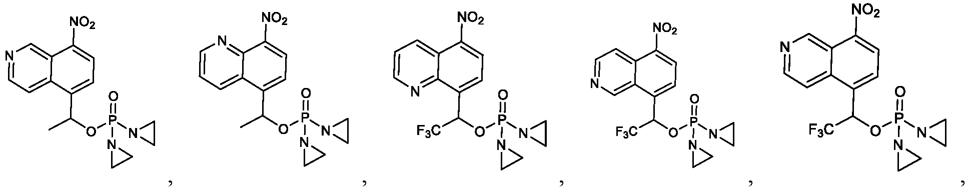

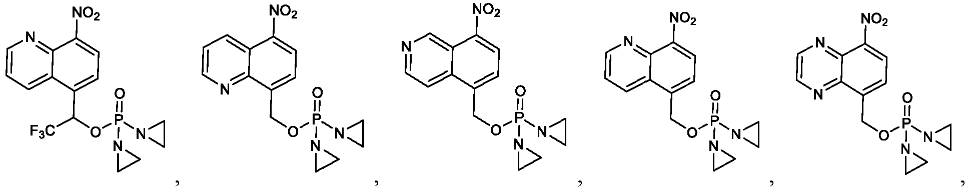

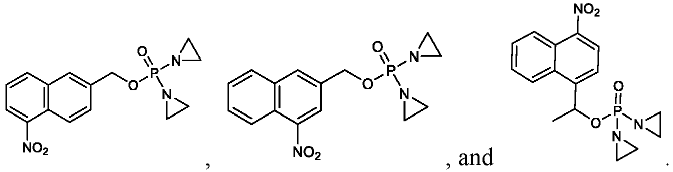

The present application also provides a pharmaceutical preparation, which is prepared from the active ingredients of the aforementioned pharmaceutical combination and pharmaceutically available excipients.

The dosage forms of the aforementioned pharmaceutical preparations are selected from granules, tablets, pills, capsules, injections, or lyophilized powder for injection.

The present application also provides the use of the aforementioned pharmaceutical combination and/or the pharmaceutical preparation in the manufacture of anti-tumor drugs.

As a preferred use of the aforementioned pharmaceutical combination and/or the aforementioned pharmaceutical preparation in the manufacture of anti-tumor drugs, the tumor is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.

The aforementioned medicaments should not only comprise a hypoxia-activated compound of formula I or II, or a salt, ester, solvate, isotopic variant, or isomer thereof, but also pharmaceutically acceptable adjuvants or excipients according to the characteristics of the drug, medicament, or preparation. The medicament may be in any dosage form for clinical administration, such as tablets, suppositories, dispersible tablets, enteric-coated tablets, chewable tablets, orally disintegrating tablets, capsules, sugar coated agents, granules, dry powders, oral solutions, small needles for injection, lyophilized powder for injection, or infusion solutions. According to the specific dosage form and administration mode, the pharmaceutically acceptable adjuvants or excipients in the medicament may include one or more of the following: such as diluents, solubilizers, disintegrants, suspensions, lubricants, adhesives, fillers, flavoring agents, sweeteners, antioxidants, surfactants, preservatives, wrappers, and pigments.

### Description of the drawings

Figure 1 shows the growth curves of tumor volume in each treatment group of mice in the PDX model of LD1-2032-361588 human ovarian cancer in vivo transplanted tumor.
Figure 2 shows the percentage changes in body weight in each treatment group of mice over time in the PDX model of LD1-2032-361588 human ovarian cancer in vivo transplanted tumor.

### Detailed Description of the Invention

The present invention will be described below with reference to specific examples. Those skilled in the art could understand that these examples are only used to illustrate the present invention and do not limit the scope of the present invention in any way.

The experimental methods in the following examples, unless otherwise specified, are all conventional methods. The raw materials of the medicaments, the reagent materials and the like used are all commercially available products unless otherwise specified.

The terms "patient" and "individual" can be used interchangeably and refer to mammals that require cancer treatment. Generally, the patient is a human. Generally, the patient is a human diagnosed with cancer. In certain embodiments, "patient" or "individual" may refer to non-human mammals (such as non-human primates, dogs, cats, rabbits, pigs, mice, or rats) used for screening, characterizing, and evaluating drugs and therapies.

A "prodrug" refers to a compound that is metabolized or otherwise converted to a compound (or drug) with biological activity or higher activity with respect to at least one property after delivery or administration. Relative to drugs, prodrugs are chemically modified in a way that makes them less active or inactive relative to the drug, but chemical modification results in the production of the corresponding drug through metabolism or other biological processes after the prodrug is administered. Prodrugs may have altered metabolic stability or delivery characteristics, fewer side effects or lower toxicity, or improved flavor relative to the active drugs. Prodrugs may be synthesized using reactants other than the corresponding drug.

"Treatment" or "treating patients" refers to delivering, using, or administering to a patient a therapeutic effective amount of medicament related to the present invention.

"Delivering", "administrating" or "using" a medicament to a patient refers to the act of directly delivering or administrating (which may be delivered or administrated by medical professionals or self-delivered or self-administered) and/or indirectly delivering or administrating, which may be the act of prescribing medicament. For example, instructing a patient to self-deliver or administer medicament and/or providing a prescription for medicament to the patient's physician is delivering or administering medicament to the patient.

The "therapeutically effective amount" of a medicament refers to the amount of the medicament that will have the desired therapeutic effects (such as alleviation, improvement, relief, or elimination of one or more clinical manifestations of cancer in the patient) when delivered to, administered to or used for a patient with cancer. The therapeutic effect does not necessarily occur by delivering or administering a single dose, and it may only occur after the deliver or administration of a series of doses. Therefore, the therapeutically effective amount may be delivered or administered in one or more times.

The "treatment" of a condition or patient refers to taking steps to achieve beneficial or desired outcomes, including clinical outcomes. For the purpose of the present invention, the beneficial or desired clinical outcomes include, but not limited to, alleviation or improvement of one or more symptoms of cancer; reduction in the severity of the disease; delay or slowing of disease progression; improvement, alleviation or stabilization of the disease state; or other beneficial outcomes. In some cases, cancer treatment may lead to partial response or stabilization of the disease.

"Tumor cells" refer to tumor cells of any appropriate species (for example, mammals, such as, murine, dogs, cats, horses, or humans).

The aforementioned description of the specific embodiments of the present invention does not limit the present invention. Those skilled in the art can make various changes or modifications according to the present invention, and any change or modification should be covered in the scope of the claims attached to the present invention as long as it do not deviate from the spirit of the present invention.

The specific experiments of the present invention are provided below.

The experimental protocol, any modification, animal welfare, and use of the animal experiments disclosed in the experimental procedures of the present invention have been reviewed and approved by the IACUC committee of the CRO (Contract Research Organization). During the experimental process, both animal welfare and experimental procedures met the requirements of AAALAC.

Compound AST is the S isomer of compound 01 in Table 1 of patent application PCT/CN2020/114519 with publication number WO2021120717A1, and also compound B in patent application PCT/CN2021/101870 with publication number WO2022052564A1, the structural formula of which is as follows:

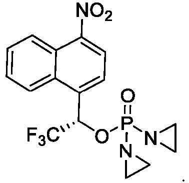

### I. Anti-tumor effects and safety evaluation of the compound TH-302, AST, and Olaparib monotherapy and a combination medicament of TH-302, AST, and Olaparib on the PDX model of LD1-2032-361588 human ovarian cancer in vivo transplanted tumor

This model was established on LD1-2032-361588 human ovarian cancer tumor tissue, and passed on to FP3+3 generation for use in this pharmacodynamic experiment. The tumor is derived from a 62-year-old female patient with ovarian cancer liver metastasis. The pathological diagnosis was poor to medium differentiated adenocarcinoma, and resistance to PARPi was developed during treatment. Through gene detection, the model is a PDX model with a pathogenic RAD51D gene mutation, and the mutation site is p.Lys91Ilefs*13.

Cut the tumor block of LD1-2032-361588 human ovarian cancer in vivo transplanted tumor into tumor tissues with a size of about 3 mm × 3 mm × 3 mm (about 45-60 mg), and inoculate them subcutaneously into NU/NU mice. Observe the mice after inoculation and monitor the growth of tumors. Group administration was performed on the 27th day of inoculation, when the average tumor volume of tumor-bearing mice was 183.67 mm³. The day of group administration was defined as day 0. The specific information on administration route, dosage, and dosage regimen is shown in Table 1.

**Table 1: Administration route, dosage, and dosage regimen**

| Group | Administration group | N | Dosage(mg/kg) | Dosage Regimen | Administration Mode |
|---|---|---|---|---|---|
| 1 | Vehicle | 6 | -- | QW×3 | i.v. |
| 2 | Olaparib | 6 | 50 | QD×32 | p.o. |
| 3 | TH-302 | 6 | 80 | QW×3 | i.v. |
| 4 | Olaparib+ TH-302 | 6 | 50+80 | QD×32+QW×3 | p.o.+i.v. |
| 5 | AST | 6 | 20 | QD×5,2 days off;2 weeks off;QD×5 | 1.v. |
| 6 | Olaparib+ AST | 6 | 50+20 | QD×32+ QD×5,2 days off;2 weeks off;QD×5 | p.o.+i.v. |

| | | | | | |
|---|---|---|---|---|---|
| Note: N: number of animals; administration volume: adjusting the administration volume according to the body weight of tumor-bearing mice (0.2 mL/20 g); p.o.: intragastric administration; i.v.: tail vein injection; QW: administering once a week; QD: administering once a day. | | | | | |

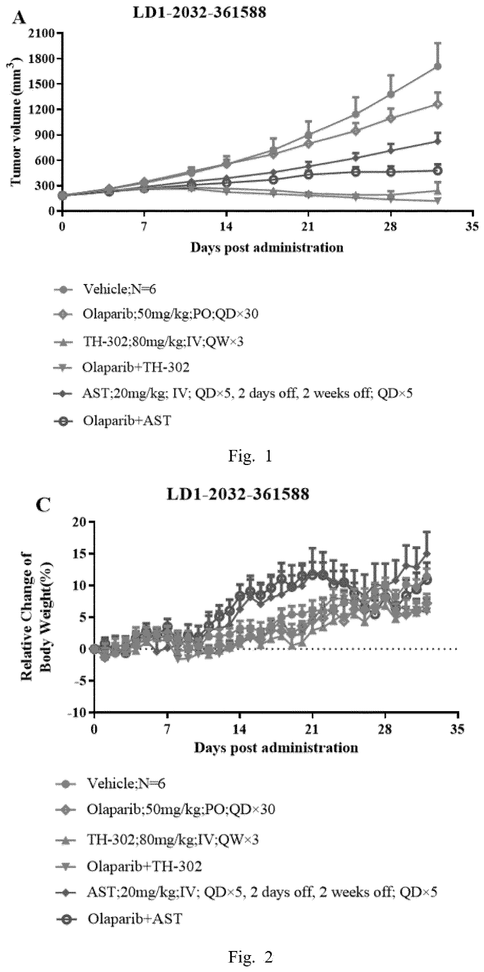

Record the tumor growth of each treatment group and control group on different days of the experiment, as shown in Table 2. Correspondingly, the growth curves of tumor volume in each group of mice are shown in Figure 1. The efficacy was evaluated based on the relative tumor proliferation rate and relative tumor suppression rate. The efficacy analysis of each group is shown in Table 3, and the significance statistical analysis is shown in Table 4. Record the weight changes of the treatment group and control group after administration, and study the safety of each group in the PDX model of human ovarian cancer in vivo transplanted tumor. The weight change results of mice are shown in Table 5, and the percentage of weight changes over time for each treatment group is shown in Figure 2 (weight change percentage: RCBW (%) = (BWᵢ - BW₀)/BW₀ × 100, BWᵢ is the weight after the start of administration, and BW₀ is the weight at the first administration).

**Table 2: Changes in tumor volume of mice in each group over treatment time (mm³)**

| Group | Days after the start of administration | | | | | | | | | |
|---|---|---|---|---|---|---|---|---|---|---|
| | 0 | 4 | 7 | 11 | 14 | 18 | 21 | 25 | 28 | 32 |
| 1 | 183.91 | 259.42 | 330.10 | 447.28 | 552.53 | 720.89 | 901.24 | 1141.20 | 1377.42 | 1709.32 |
| 2 | 183.92 | 265.96 | 343.35 | 468.60 | 555.15 | 670.34 | 794.58 | 945.81 | 1095.13 | 1261.91 |
| 3 | 183.34 | 244.75 | 265.86 | 277.14 | 264.67 | 243.36 | 208.86 | 190.63 | 192.06 | 239.28 |
| | | | | | | | | | | |
| 4 | 183.92 | 237.12 | 259.06 | 263.96 | 222.58 | 200.03 | 184.19 | 158.59 | 135.09 | 118.07 |
| 5 | 183.46 | 231.94 | 284.27 | 349.92 | 388.25 | 457.15 | 528.03 | 625.61 | 712.90 | 823.26 |
| 6 | 183.49 | 228.95 | 267.35 | 306.27 | 333.34 | 369.47 | 429.87 | 461.81 | 461.94 | 476.89 |

Tumor volume and weight measurements of tumor-bearing mice are as follows: measuring with a vernier caliper twice a week. The calculation formula of tumor volume is V = 0.5 a × b², where a and b represent the length and width of the tumor, respectively.

**Table 3: Summary of anti-tumor efficacy evaluation of test compounds in LD1-2032-361588 human ovarian cancer subcutaneous xenograft tumor model**

| **Group** | **Treatment group** | **Tumor volume /Day 0** | **Tumor volume /Day 32** | **Relative tumor volume /Day 32** | **T/C (%) /Day 32** | **TGI(% ) /Day 32** | **Tumor weight (g) /Day 32** |
|---|---|---|---|---|---|---|---|
| 1 | Vehicle | 183.91±15.42 | 1709.32±271.8 0 | 9.29 | -- | -- | 1.546±0.24 6 |
| 2 | Olaparib | 183.92±14.84 | 1261.91±136.4 3 | 6.86 | 73.82 | 29.33 | 1.202±0.11 4 |
| 3 | TH-302 | 183.34±10.76 | 239.28±101.70 | 1.31 | 14.04 | 96.33 | 0.219±0.11 7 |
| 4 | Olaparib+ TH-302 | 183.92±16.15 | 118.07±25.29 | 0.64 | 6.91 | 104.32 | 0.083±0.01 6 |
| 5 | AST | 183.46±15.86 | 823.26±100.46 | 4.49 | 48.28 | 58.06 | 0.730±0.09 2 |
| 6 | Olaparib+ AST | 183.49±11.85 | 476.89±73.20 | 2.60 | 27.96 | 80.77 | 0.389±0.07 1 |

The relative tumor proliferation rate T/C (%) is calculated as follows: T/C % = T_{RTV}/C_{RTV} × 100% (T_{RTV}: treatment group RTV; C_{RTV}: negative control group RTV. The relative tumor volume (RTV) was calculated according to the results of tumor measurements, and the calculation formula is RTV=Vt / V₀, wherein, V₀ is the average tumor volume measured during group administration (i.e. d₀), Vₜ is the average tumor volume at a certain measurement, and T_{RTV} and C_{RTV} take the data from the same day.

The tumor growth inhibition rate TGI (%) = [1- (Tᵢ - T₀) / (Vᵢ - V₀)] × 100, where Tᵢ is the average tumor volume of the compound group after the start of administration, T₀ is the average tumor volume of the compound group at the first administration, V₀ is the average tumor volume of the solvent control group at the first administration, and Vᵢ is the average tumor volume of the solvent control group after the start administration.

**Table 4: Statistical Analysis**

| **Group** | **Treatment group** | **P value vs vehicle (TV)** | **P value vs TH-302 (TV)** | **P value vs AST (TV)** |
|---|---|---|---|---|
| 1 | Vehicle | -- | -- | -- |
| 2 | Olaparib | 0.1195 | -- | -- |
| 3 | TH-302 | <0.0001 | -- | -- |
| 4 | Olaparib+ TH-302 | <0.0001 | 0.2744 | -- |
| 5 | AST | 0.0005 | -- | -- |
| 6 | Olaparib+ AST | <0.0001 | -- | 0.0192 |

**Table 5: Changes in mouse body weight (g) over treatment time**

| Group | Days after the start of administration | | | | | | | | | | | | | | | | |
|---|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | 15 | 16 |
| 1 | 23.87 | 23.56 | 23.69 | 23.95 | 24.43 | 24.47 | 24.39 | 24.25 | 24.26 | 24.28 | 23.95 | 24.33 | 24.34 | 24.41 | 24.64 | 24.65 | 24.54 |
| 2 | 23.44 | 23.14 | 23.39 | 23.42 | 23.56 | 23.87 | 24.05 | 24.03 | 23.72 | 23.51 | 23.63 | 23.38 | 23.59 | 23.50 | 23.73 | 23.92 | 23.79 |
| 3 | 24.05 | 23.75 | 23.94 | 23.91 | 23.98 | 24.30 | 24.43 | 24.57 | 23.95 | 24.03 | 23.97 | 23.81 | 23.97 | 24.07 | 24.22 | 24.55 | 24.18 |
| 4 | 24.00 | 23.93 | 24.10 | 24.10 | 24.21 | 24.59 | 24.33 | 24.33 | 23.62 | 23.65 | 23.81 | 23.87 | 23.81 | 24.08 | 24.25 | 24.35 | 24.47 |
| 5 | 23.60 | 23.65 | 23.68 | 23.71 | 23.79 | 23.89 | 23.48 | 23.61 | 23.69 | 23.91 | 23.81 | 24.24 | 24.30 | 24.45 | 25.03 | 25.45 | 25.20 |
| 6 | 23.72 | 23.93 | 23.65 | 23.58 | 24.13 | 24.40 | 24.24 | 24.55 | 24.01 | 24.24 | 24.16 | 24.57 | 24.92 | 25.13 | 25.68 | 25.85 | 25.72 |

| Group | Days after the start of administration | | | | | | | | | | | | | | | | |
|---|---|---|---|---|---|---|---|---|---|---|---|---|---|---|---|---|---|
| | 17 | 18 | 19 | 20 | 21 | 22 | 23 | 24 | 25 | 26 | 27 | 28 | 29 | 30 | 31 | 32 | |
| 1 | 24.73 | 25.05 | 25.16 | 25.18 | 25.33 | 25.29 | 25.38 | 25.62 | 25.59 | 25.36 | 26.05 | 26.26 | 25.83 | 26.29 | 26.20 | 26.60 | |
| 2 | 24.12 | 24.09 | 23.98 | 24.29 | 24.48 | 24.57 | 24.83 | 24.47 | 24.90 | 24.85 | 24.99 | 25.36 | 24.76 | 24.72 | 24.82 | 25.13 | |
| 3 | 24.38 | 24.49 | 24.12 | 24.25 | 24.72 | 24.84 | 25.09 | 25.35 | 25.28 | 25.01 | 25.49 | 25.64 | 25.15 | 25.39 | 25.38 | 25.54 | |
| 4 | 24.67 | 25.07 | 24.48 | 24.56 | 25.08 | 25.55 | 25.90 | 25.89 | 25.79 | 25.88 | 25.96 | 26.13 | 25.65 | 25.49 | 25.47 | 25.42 | |
| 5 | 25.45 | 25.55 | 25.79 | 25.87 | 26.38 | 26.29 | 26.05 | 26.00 | 25.77 | 25.51 | 25.94 | 25.86 | 26.08 | 26.63 | 26.57 | 27.07 | |
| 6 | 25.99 | 26.32 | 26.04 | 26.43 | 26.48 | 26.47 | 26.10 | 26.18 | 25.79 | 25.26 | 25.01 | 25.65 | 25.23 | 25.69 | 25.93 | 26.28 | |

On Day 32 after the start of administration, the average tumor volume of tumor-bearing mice of the control group was 1709.32 ± 271.80 mm³. The average tumor volumes of tumor-bearing mice of the test compound Olaparib (50 mg/kg, PO, QD * 32days) monotherapy group, TH-302 (80 mg/kg, IV, QW × 3) monotherapy group, Olaparib combined with TH-302 administration group, AST (20 mg/kg, IV, QD×5, 2 days off; 2weeks off; QD × 5) monotherapy group and Olaparib combined with AST administration group were 1261.91 ± 136.43 mm³, 239.28 ± 101.70 mm³, 118.07 ± 25.29 mm³, 823.26 ± 100.46 mm³, and 476.89 ± 73.20 mm³, respectively; the relative tumor proliferation rates T/C (%) were 73.82%, 14.04%, 6.91%, 42.28%, and 27.96%, respectively; and the tumor volume growth inhibition rates TGI (%) were 29.33%, 96.33%, 104.32%, 58.06%, and 80.77%, respectively.

The experimental results showed that, in the human ovarian cancer tumor model with pathogenic RAD51D gene mutation, the test compound Olaparib monotherapy group had poor tumor suppression effect, indicating that the tumor model was indeed resistant to Olaparib. Compared with the control group, the test compound TH-302 monotherapy group, Olaparib combined with TH-302 administration group, AST monotherapy group, and Olaparib combined with AST administration group all showed statistically significant inhibitory effects on tumor growth (p<0.05 and T/C%<40%). There was no statistically significant difference between the Olaparib combined with TH-302 group and the TH-302 monotherapy group. However, there was a statistically significant difference between the Olaparib combined with AST group and the AST monotherapy group, indicating that the combination of AST and Olaparib can improve its inhibitory effect on tumor growth to a certain extent.

The body weight of experimental animals was used as an indirect indicator to monitor drug toxicity. After administration, no significant weight loss was observed in the mice of each treatment group, indicating that tumor-bearing mice had good tolerance to the treatment of various drugs at the experimental dose.

In clinical studies, researchers use PARP inhibitors to treat patients with homologous recombination repair deficiencies. However, with the clinical application of PARPi, PARPi resistance has become an inevitable problem in its clinical application. The existing researches indicate that homologous recombination repair, DNA replication fork protection, and changes in PARPi pharmacokinetics are the main causes of PARPi resistance. The action principle of TH-302 and AST is completely different from that of PARPi, but they are equally effective for patients with homologous recombination repair deficiencies (pathogenic RAD51D gene mutation being one of the homologous recombination repair deficiency mutations). This provides a new treatment option for patients with PARPi resistance and homologous recombination repair deficiency mutations in clinical practice.

Our research results also indicate that TH-302 and AST have good tumor inhibitory ability in the Olaparib resistant mouse ovarian cancer PDX model, and compared to AST monotherapy, the combination of AST and Olaparib has a significant synergistic effect. This brings new hope for the clinical treatment of patients with pathogenic HRR gene mutations but PARPi resistance, and finds important theoretical and experimental evidence.

TH-302 and AST are small molecule prodrugs that can be activated under oxygen deficiency/hypoxic conditions and release cell toxins, thereby specifically killing tumor cells and tissues. TH-302 and AST are hypoxia-activated DNA alkylating agents.

The compounds with general formula of the following formula I: 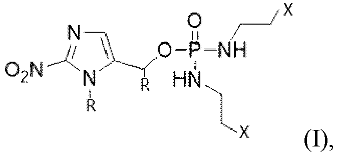 have been proven to have a mechanism similar to that of TH-302 in relevant patent applications; thus, it is entirely predictable that such compounds with the general formula have similar effects as TH-302.

Similarly, the compounds with general formula of the following formula II: 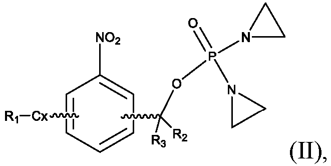 have also been proven to have a mechanism similar to that of AST in the relevant patent application PCT/CN2020/114519 (publication number WO2021120717A1); thus, it is completely predictable that such compounds with the general formula have similar effects as AST.

Although the PARP inhibitor selected in the embodiments of the present application is Olaparib, Rucaparib, Niraparib, Talazoparib, Fluzoparib, Pamiparib and the like are also belong to PARP inhibitors. Their mechanism of action is similar to that of Olaparib, both of which block the enzymes involved in repairing impaired DNA from functioning. Therefore, it can be inferred that PARPi such as Rucaparib, Niraparib, Talazoparib, Fluzoparib, and Pamiparib have similar tumor suppression effects as Olaparib in the aforementioned experiments.

## Claims

1. A treatment method for treating patients with cancer or tumors with impaired DNA repair enzymes, comprising administering a hypoxia-activated compound, or a salt, ester, solvate, isotopic variant, or isomer thereof either as monotherapy or in combination with other drugs.

2. The treatment method according to claim 1, wherein the DNA repair enzyme impairment is selected from defects in one or more of the following gene: BRCA1, BRCA2, FANCA, FANCD1, FANCD2, ATM, ATR, CHEK1, CHEK2, CTP, BARD1, BRIP1, PALB2, RAD51, RAD52, RAD54, RAD55, RAD57, FAM175, NBN, Rad50, MRE11, p53, NBS1, XRS2, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA Ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RBBP8, SMC5, USP11, and WRN; the RAD51 is selected from XRCC2, XRCC3, RAD51B, RAD51C, and RAD51D, preferably RAD51D.

3. The treatment method according to claim 1, wherein,
the other drugs include a PARP inhibitor which is selected from Olaparib, Rucaparib, Niraparib, Talazoparib, Fluzoparib, and Pamiparib;
the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.

4. The treatment method according to claim 3, wherein the patient is resistant or insensitive to the PARP inhibitor.

5. A treatment method for treating patients with cancer or tumors caused by RAD51D gene defects, comprising administering a hypoxia-activated compound, or a salt, ester, solvate, isotopic variant, or isomer thereof as monotherapy, wherein,
the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

6. A treatment method for treating patients with cancer or tumors caused by RAD51D gene defects, comprising administering a hypoxia-activated compound, or a salt, ester, solvate, isotopic variant, or isomer thereof in combination with PARP inhibitors, wherein,
the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer;
the PARP inhibitor is selected from Olaparib, Rucaparib, Niraparib, Talazoparib, Fluzoparib, and Pamiparib.

7. The treatment method according to claim 6, wherein the patient is resistant or insensitive to the PARP inhibitor.

8. The treatment method according to any one of claims 1-7, wherein the hypoxia-activated compound is selected from the compounds of the following formula I or II: 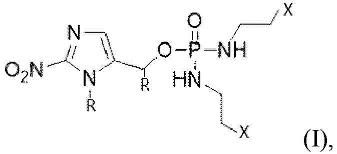
wherein, Rs are each independently selected from H-, CH₃, and -CH₂CH₃, and Xs are each independently selected from leaving functional groups such as Cl, Br, MsO, and TsO; 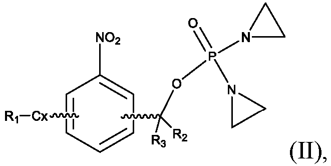
wherein, the definitions of R₁, R₂, R₃, and Cₓ are as described in the claims of the patent application PCT/CN2020/114519 with publication number WO2021120717A1.

9. The treatment method according to claim 8, wherein,
the hypoxia-activated compound of formula I is selected from the compounds of the following structures: 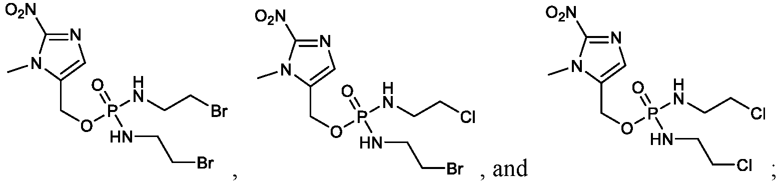
the hypoxia-activated compound of formula II is selected from the compounds of the following structures:

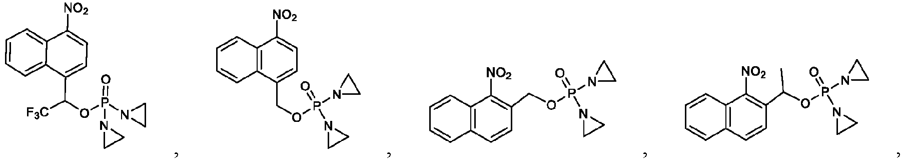

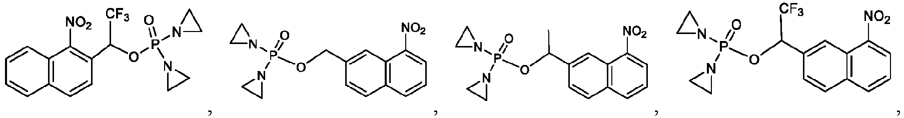

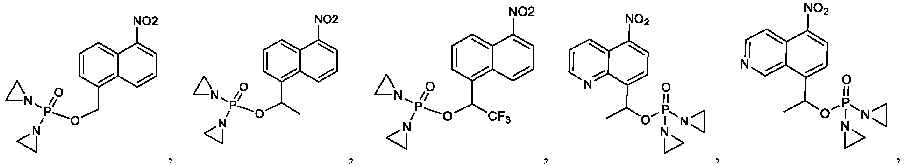

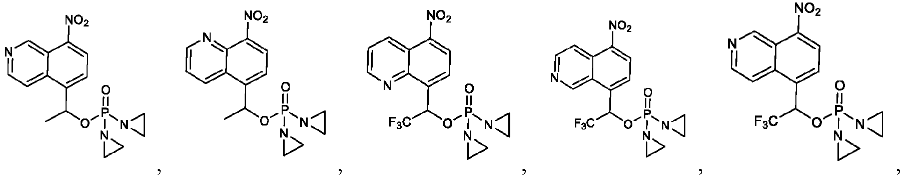

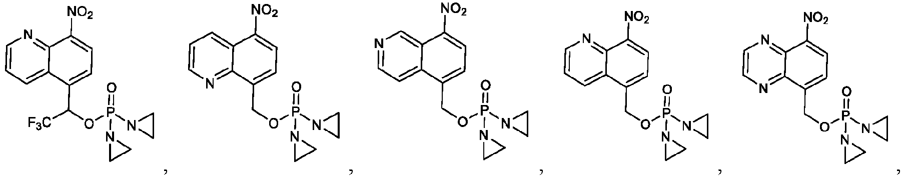

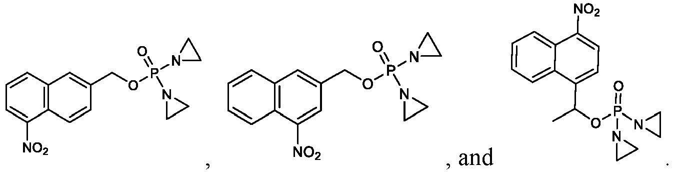

10. A treatment method, comprising the following steps:
detecting RAD51D gene mutations in patients with cancer or tumors;
if the patient or the patient's tumor tissue has a pathogenic RAD51D gene mutation, administering a medicament containing a hypoxia-activated compound of formula I or II either as monotherapy or in combination with a PARP inhibitor for treatment: 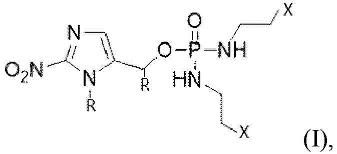
wherein, Rs are each independently selected from H-, CH₃, and -CH₂CH₃, and Xs are each independently selected from leaving functional groups such as Cl, Br, MsO, and TsO; 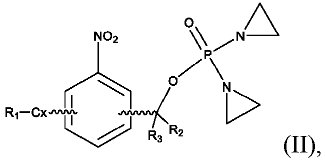
wherein, the definitions of R₁, R₂, R₃, and Cₓ are as described in the claims of patent application PCT/CN2020/114519 with publication number WO2021120717A1.

11. The treatment method according to claim 10, wherein,
the PARP inhibitor is selected from Olaparib, Rucaparib, Niraparib, Talazoparib, Fluzoparib, and Pamiparib.

12. The treatment method according to claim 10 or 11, wherein the patient is resistant or insensitive to the PARP inhibitor.

13. Use of a hypoxia-activated compound of the following formula I or II, or a salt, ester, solvate, isotopic variant, or isomer thereof in the manufacture of a medicament for treating patients with cancer or tumors with pathogenic RAD51D gene mutations either as monotherapy or in combination with other therapeutic drugs: 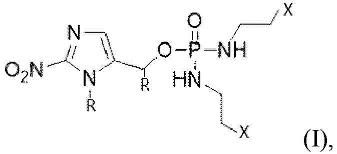
wherein, Rs are each independently selected from H-, CH₃, and -CH₂CH₃, and Xs are each independently selected from leaving functional groups such as Cl, Br, MsO, and TsO; 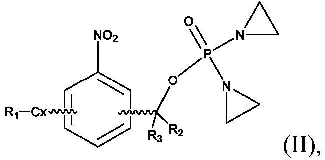
wherein, the definitions of R₁, R₂, R₃, and Cₓ are as described in the claims of patent application PCT/CN2020/114519 with publication number WO2021120717A1.

14. The use according to claim 13, wherein,
the other therapeutic drugs include a PARP inhibitor which is selected from Olaparib, Rucaparib, Niraparib, Talazoparib, Fluzoparib, and Pamiparib;
the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

15. The use according to claim 13 or 14, wherein the patient is resistant or insensitive to the PARP inhibitor.

16. A medicament for treating the cancer caused by pathogenic RAD51D gene mutation, wherein the medicament contains a hypoxia-activated compound of the following formula I or II, or a salt, ester, solvate, isotopic variant, or isomer thereof: 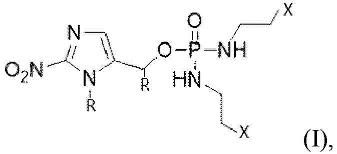
wherein, Rs are each independently selected from H-, CH₃, and -CH₂CH₃, and Xs are each independently selected from leaving functional groups such as Cl, Br, MsO, and TsO; 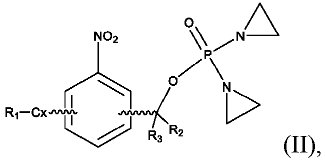
wherein, the definitions of R₁, R₂, R₃, and Cₓ are as described in the claims of patent application PCT/CN2020/114519 with publication number WO2021120717A1.

17. The medicament according to claim 16, the medicament is used in combination with a PARP inhibitor, wherein,
the PARP inhibitor is selected from Olaparib, Rucaparib, Niraparib, Talazoparib, Fluzoparib, and Pamiparib;
the cancers is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer.

18. The medicament according to claim 17, wherein the patient is resistant or insensitive to the PARP inhibitor.

19. A pharmaceutical combination, **characterized in** comprising the following substances as active ingredients:
a hypoxia-activated compound, or a salt, ester, solvate, isotopic variant, or isomer thereof, and PARP inhibitors;
wherein, the active ingredients are formulated together or separately for use in compatible combination simultaneously or separately.

20. The pharmaceutical combination according to claim 19, **characterized in that** the pharmaceutical combination is used for treating patients with cancer or tumors with impaired DNA repair enzyme.

21. The treatment method according to claim 20, wherein,
the DNA repair enzyme impairment is selected from one or more of the following gene defects: BRCA1, BRCA2, FANCA, FANCD1, FANCD2, ATM, ATR, CHEK1, CHEK2, CTP, BARD1, BRIP1, PALB2, RAD51, RAD52, RAD54, RAD55, RAD57, FAM175, NBN, Rad50, MRE11, p53, NBS1, XRS2, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA Ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RBBP8, SMC5, USP11, and WRN; the RAD51 is selected from XRCC2, XRCC3, RAD51B, RAD51C, and RAD51D, preferably RAD51D;
the cancer is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.

22. The pharmaceutical combination according to claim 19, wherein,
the PARP inhibitor is selected from Olaparib, Rucaparib, Niraparib, Talazoparib, Fluzoparib, and Pamiparib;
the hypoxia-activated compound is selected from the compounds of the following formula I or II: 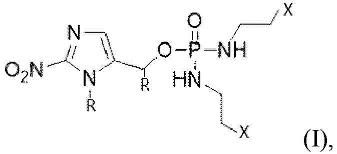
wherein, Rs are each independently selected from H-, CH₃, and -CH₂CH₃, and Xs are each independently selected from leaving functional groups such as Cl, Br, MsO, and TsO; 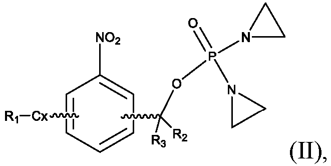
wherein, the definitions of R₁, R₂, R₃, and Cₓ are as described in the claims of patent application PCT/CN2020/114519 with publication number WO2021120717A1.

23. The pharmaceutical combination according to claim 22, wherein,
the hypoxia-activated compound of formula I is selected from the compounds of the following structures: 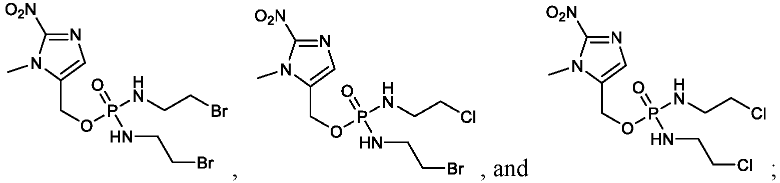
the hypoxia-activated compound of formula II is selected from the compounds of the following structures:

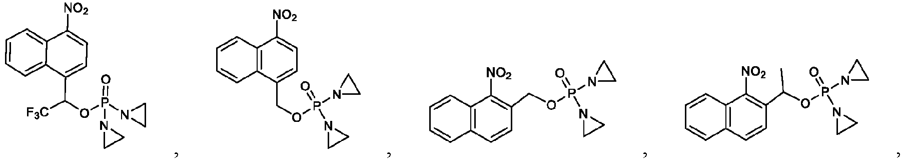

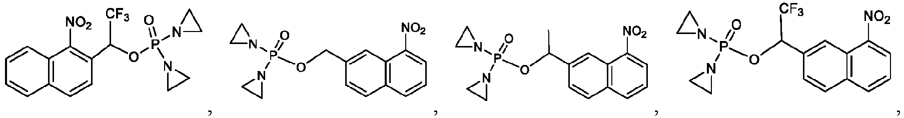

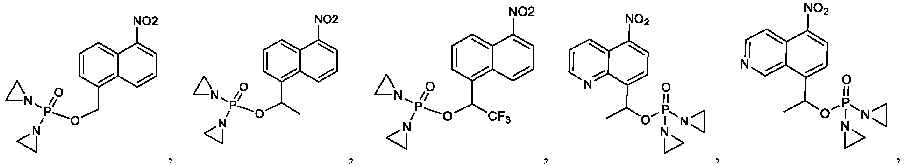

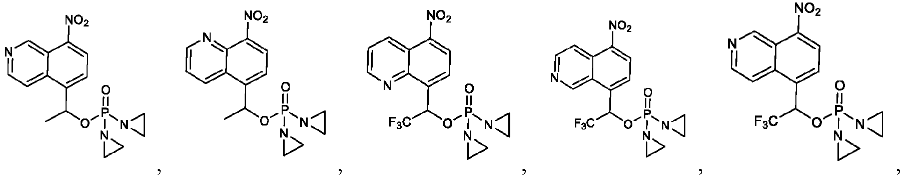

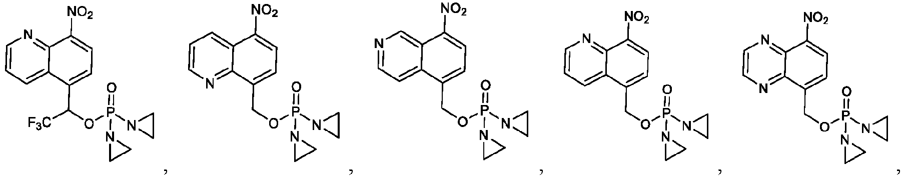

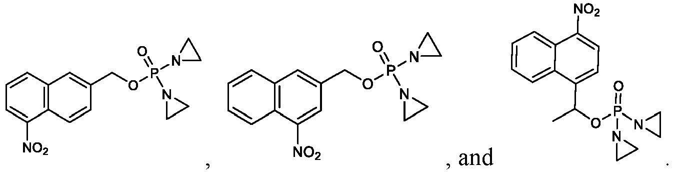

24. A pharmaceutical preparation, **characterized in that** it is prepared from the active ingredients of the pharmaceutical combination according to claim 19 and pharmaceutically acceptable excipients.

25. The pharmaceutical preparation according to claim 24, **characterized in that** the pharmaceutical preparation is in a dosage form selected from granules, tablets, pills, capsules, injections, or lyophilized powder for injection.

26. Use of the pharmaceutical combination according to claim 19 and/or the pharmaceutical preparation according to claim 24 in the manufacture of an anti-tumor drug.

27. The use according to claim 26, wherein the tumor is selected from ovarian cancer, breast cancer, pancreatic cancer, fallopian tube cancer, primary peritoneal cancer, gastric cancer, prostate cancer, liver cancer, colon cancer, rectal cancer, lung cancer, and bladder cancer, wherein the lung cancer is preferably non-small cell lung cancer, small cell lung cancer.
